# EUROPEAN PATENT SPECIFICATION

(11) **EP 2 276 426 B1**
(45) Date of publication and mention of the grant of the patent: **21.09.2016**
(21) Application number: 09717342.1
(22) Date of filing: 03.03.2009
(51) Int. Cl.: A61F 2/32, A61B 17/68, A61B 17/74, A61F 2/30, A61F 2/36

(54) **SYSTEM FOR PERFORMING A MODULAR REVISION HIP PROSTHESISS**
SYSTEM ZUR DURCHFÜHRUNG EINER MODULAREN HÜFTPROTHESENREVISION
SYSTÈME POUR RÉALISER UNE PROTHÈSE DE LA HANCHE À TIGE DE RÉVISION MODULAIRE

(30) Priority: 03.03.2008 US 33213 P
(43) Date of publication of application: 26.01.2011
(73) Proprietor: Smith & Nephew, Inc., Memphis, TN 38116 (US)
(72) Inventor: BERGIN, Alisha, W., Southaven Mississippi 38671 (US); LAMBERT, Richard, D., Germantown Tennessee 38139 (US); KELMAN, David, C., Collierville Tennessee 38017 (US)
(74) Representative: Smith & Nephew
(86) International application number: PCT/US2009/035857
(87) International publication number: WO 2009/111459

(56) References cited:
- EP-A2- 1 205 161
- EP-A2- 1 493 407
- US-A- 4 549 319
- US-A- 4 883 491
- US-A- 4 944 759
- US-A- 5 906 644
- US-A1- 2004 010 319
- US-B2- 7 122 056

## Description

### Field of the Invention

This invention relates generally to a system for implanting a revision hip prosthetic and, more particularly, to a system for implanting a modular revision hip prosthetic. EP 1 205 161 A2 discloses a revision hip important comprising a stem, a sleeve and a neck which is not configured to be received in the stem.

### Summary of the Invention

It is in view of the above problems that the present invention was developed.

An embodiment of a revision hip implant comprises a stem, a sleeve, and a neck. The stem has a proximal body portion, a proximal intramedullary canal portion and a distal intramedullary canal portion. The proximal intramedullary canal portion and the distal intramedullary canal portion are generally aligned along a long axis of the stem. The proximal body portion is proximally oriented relative to the proximal intramedullary canal portion and further has a surface extending generally radially out from the long axis of the stem. The proximal intramedullary canal portion has a distally extending taper surface. The sleeve is configured to be received over the distal intramedullary canal portion of the stem and further has an inner surface configured to mate with the distally extending taper surface of the proximal intramedullary canal portion. The neck is configured to be received in the proximal body portion of the stem. The proximal body portion of the stem has a neck receiving surface configured to mate with a neck taper portion of the neck. The neck taper portion is oriented at an angle relative to the long axis. The sleeve further comprises a conical portion and a cylindrical portion. The conical portion is oriented proximal to the proximal stem body portion and the cylindrical portion extends distally away from the conical portion.

According to another embodiment of the invention, the distal intramedullary portion of the stem further comprises flutes extending along the length of the stem.

Alternatively, the surface extending generally radially out from the long axis of the stem on the body portion of the stem may be configured to rest upon a bone cut.

In another embodiment, the sleeve is configured to asymmetrically extend away from the long axis of the stem.

According to another embodiment, the neck is received on the stem along a first axis. A head is received on the neck along a second axis, the first and second axes being parallel to each other.

Further features, aspects, and advantages of the present invention, as well as the structure and operation of various embodiments of the present invention, are described in detail below with reference to the accompanying drawings.

### Brief Description of the Drawings

The accompanying drawings, which are incorporated in and form a part of the specification, illustrate embodiments of the present invention and together with the description, serve to explain the principles of the invention. In the drawings:
Figure 1 is a view of an embodiment of a revision hip implant set including a group of stems, a group of sleeves, and a group of necks;
Figure 2 is an exploded view of an embodiment of a hip implant according to an aspect of the invention;
Figure 3 is a view of an embodiment of a revision hip implant according to an aspect of the invention;
Figure 4 is a cutaway view of a femur with distal preparation instrumentation according to an aspect of the invention;
Figure 5 is a cutaway view of the femur with trialing instrumentation according to an aspect of the invention;
Figure 6 is a cutaway view of the femur with additional trialing instrumentation according to an aspect of the invention;
Figure 7 is a cutaway view of the femur with proximal preparation instrumentation according to an aspect of the invention;
Figure 8 is a cutaway view of the femur with portions of a hip implant and implant instrumentation; and
Figures 9 through 11 are cutaway views of the femur with portions of a hip implant and hip instrumentation according to an aspect of the invention.

### Detailed Description of the Embodiments

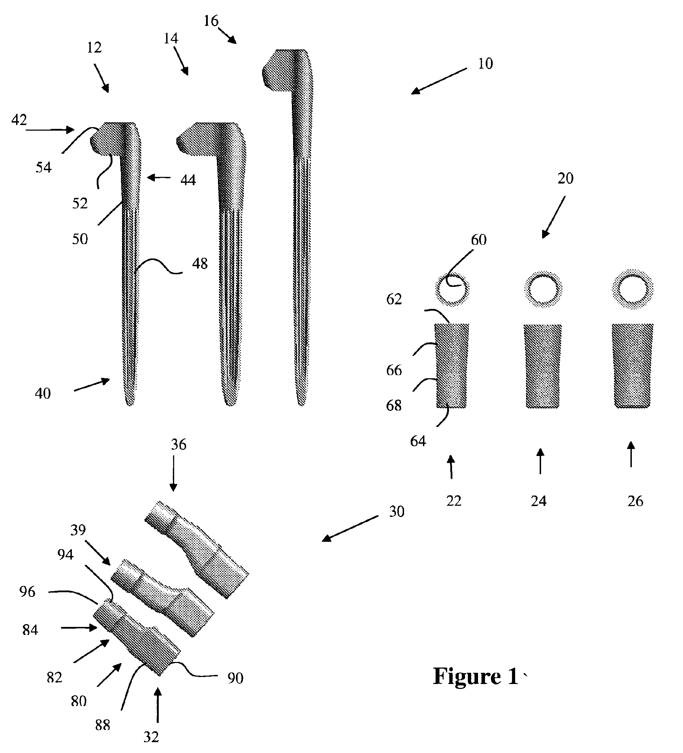

Referring to the accompanying drawings in which like reference numbers indicate like elements, Figure 1 illustrates a view of an embodiment of a revision hip implant set including a group of stems 10, a group of sleeves 20, and a group of necks 30. The set includes a three piece modular implant system that may allow a surgeon to address the individual patient needs. The set may be used in revision hip arthroplasty where other treatments or devices have failed in rehabilitating hips damaged as a result of trauma or noninflamatory degenerative joint disease (NIDJD) or any of its composite diagnoses of osteoarthritis, avascular necrosis, traumatic arthritis, slipped capital epiphysis, fused hip, fracture of the pelvis and diastrophic variant.

Modular hip components may also be indicated for inflammatory degenerative joint disease including rheumatoid arthritis, arthritis secondary to a variety of diseases and anomalies and congenital dysplasia. Other indications include old, remote osteomyelitis with an extended drainage-free period, in which case the patient should be warned of an above normal danger of infection postoperatively. Such a modular revision system may also be used as treatments of non-union, femoral neck fracture and trochanteric fractures of the proximal femur with head involvement that are unmanageable using other techniques, endoprosthesis, femoral osteotomy or Girdlestone resection, fracture-dislocation of the hip, and correction of deformity. The three piece implant system may provide distal fixation and secondary proximal support while allowing the surgeon intra-operative flexibility to achieve the best patient fit. Better fixation and fit may provide better implant stability and outcomes.

Preoperative planning for a revision total hip arthroplasty should require a standard set of radiographs, which includes an antero-posterior (A-P) radiograph of the pelvis and a lateral radiograph of the affected hip. Depending on the length of the existing femoral component several additional radiographs may be necessary. Specifically, the A-P and lateral radiographs should include the entire femoral component. On occasion a full-length A-P radiograph of the entire femur may be necessary. As part of the preoperative work-up, the surgeon may consider other imaging modalities such as bone scans and computerized tomography (CT). However, these are not typically necessary for preoperative templating. Determine the appropriate classification for the femoral revision, for example the Paprosky Revision Classification. This will aid in determining the appropriate position and size of the revision stem you will need. As with primary THA preop planning, establishing proper leg length requires assessment of a number of clinical and radiographic parameters. Establishing the proper reference lines requires using a horizontal line between the inferior portion of the teardrop as well as a horizontal line between the inferior margin of the obtruator foramen and ischial tuberosity. Due to the often distorted anatomy in revision cases, utilizing all three reference lines may be necessary.

Similarly, due to bony defects on the femoral side, a combination of anatomic landmarks such as the superior margin of the greater trochanter and inferior margin of the lesser trochanter must be utilized. These obviously need to be compared to similar points in the contralateral side using the A-P radiograph. Any pelvic obliquities and/or spinal deformity must also be taken into account based on radiographic and clinical assessments. The consideration of all relevant factors is necessary to successfully restore the patient's proper leg length.

A revision hip implant according to an aspect of the invention generally includes a stem 12, 14, or 16, a sleeve 22, 24, or 26, and a neck 32, 34, or 36. The stem may be chosen from the group of stems 10 and similarly the sleeve and neck may be chosen from the group of sleeves 20 and the group of necks 30, respectively.

The stem 12, 14, or 16 may be a tapered stem having a distal intramedullary portion 40, a proximal portion 42 and a proximal intramedullary portion 44. The distal portion 40 may include flutes 48 positioned distally for improved fixation of the implant to the diaphysis. The diameters and lengths of the distal portion 40 of the stem may vary according to patient need. The proximal portion 42 includes a body having a distal flat surface 52 and a neck interface surface 54. The distal flat surface 52 may be oriented to rest upon a cut portion of a femur. The neck interface surface 54 is configured to receive a neck. The proximal intramedullary portion 44 of the stem includes a sleeve mating surface 50. The sleeve mating surface 50 receives one of the group of sleeves 20.

The sleeves 20 in the system provide secondary proximal support to the distal fixation and enhance implant stability. The sleeves may be conical sleeves and may be coated, such as coated with Smith & Nephew's STIKTITE™ coating and then coated in hydroxyapatite (HA). The sleeves 20 include an internal mating surface 60 a proximal end portion 62, a distal end portion 64, a conical portion 66 and a cylindrical portion 68. Generally, the proximal end portion 62 of the sleeve is larger than the distal end portion 64.

Different sleeves 22, 24, and 26 may have different relationships between the different portions of the sleeves. For example, a first sleeve may have a smaller internal mating surface 60 (for a smaller diameter stem portion) but have a larger conical portion 66, thus making the sleeve thicker. The shape and characteristics of the sleeve are chosen based upon the bone of the patient. Where thicker sleeves are necessary to ensure proximal fixation, a thicker sleeve may be used. Where sleeves requiring more taper are necessary, a sleeve with a larger proximal end portion 62 relative to the proximal end portion 64 may be used.

Alternatively, different sleeves may be asymmetric. Such a sleeve may extend more medially, more anteriorly, more laterally, or more posteriorly relative to the long axis of the stem. Such a sleeve may allow the surgeon to plan for additional asymmetric bone defects within the IM canal.

Necks 32, 34, and 36 for the modular stem may be modular and available in multiple offset, leg length and version options. For example, the offsets and leg lengths may include a standard offset, high offset, and a high offset + 10mm. The version may include anteverted left and anteverted right. This allows the surgeon to fine tune the offset, length and version to achieve fit and function for each patient.

In order to achieve different options in the neck, the neck includes a stem mating portion 80 a neck extension 82 and a head mating portion 84. A stem taper 88 is used to join the neck to the stem. A distal end 90 of the neck is inserted within the neck mating surface 54. A head taper 94 aligns with a proximal end portion 96 of the neck. The proximal end portion 96 defines the most proximal portion of the neck, which is used to help determine offset, leg length and version. The head taper 94 joins the neck to a femoral head, thus setting the offset, version, and length of the prosthesis relative to an acetabulum.

Each neck is preferably made of cobalt chrome and includes the circulotrapezoidal neck design for increased range of motion. The 12/14 taper of the neck accepts compatible heads. The geometries of the necks may be changed in order to achieve different implant configurations. Neutral necks (neutral 0° anteversion) may be available in standard and high offset. The standard neutral neck may have a neck shaft angle of 131°. The high offset neutral neck may have a neck shaft angle of 125° when inserted in the varus orientation and 137° when inserted in the valgus orientation. Left anteverted necks may shift the femoral head, 10 degrees, anteriorly relative to the stem on a left hip and posteriorly relative to the stem on a right hip when inserted in the varus orientation. Right anteverted necks may shift the femoral head center based on the operative leg in an equivalent manner. Anteverted necks may be available in one offset option, for example 6mm more than the standard offset neck, and then may create a neck shaft angle of 125° when inserted in the varus orientation and a neck shaft angle of 137° when inserted in the valgus orientation. The high offset +10 neck may have a neck shaft angle similar to a high offset neck and an additional 10mm of height.

To aid in proper neck orientation, a marking such as a laser etch on top of the neck trunnion (the proximal neck portion 96) is provided on the high offset and anteverted neck implants and plastic neck trials. Such markings may be arrows or other indicia such that when the neck is inserted in the pocket on the neck mating surface, the arrow on top of the neck points superiorly relative to the patient's femur if the neck shaft angle is varus, 125° and inferiorly if the neck shaft angle is valgus, 137°. To reduce intraoperative confusion of left & right anteverted necks, the top of the trunnion of the necks may also be laser etched with an "L" for left anteverted and an "R" for right anteverted. Additionally, color coding of the anteverted neck implant packaging and corresponding trial necks may be provided to account for neck characteristics or neck orientations.

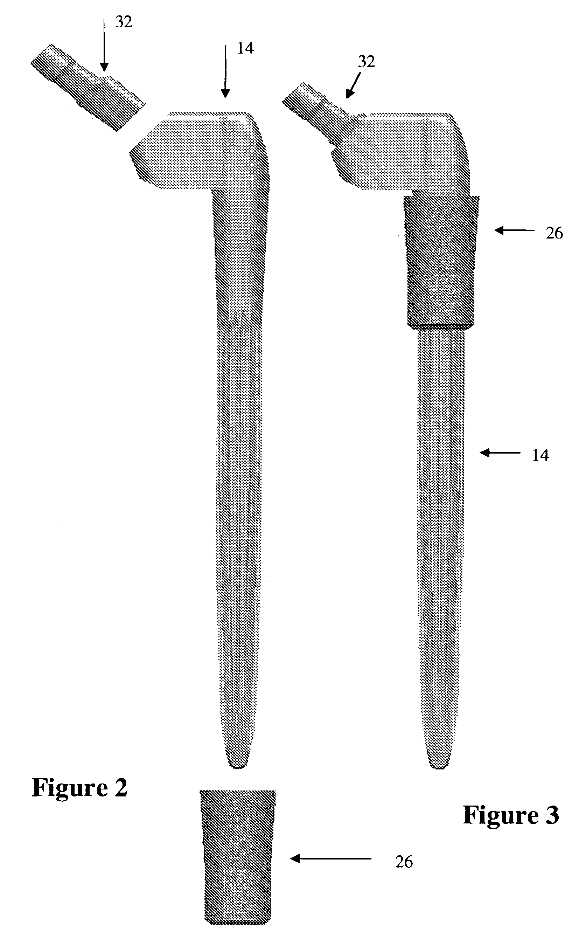

Turning now to Figures 2 and 3, Figure 2 is an exploded view of an embodiment of a hip implant according to an aspect of the invention and Figure 3 is a view of an embodiment of a revision hip implant according to an aspect of the invention. The sleeve 26 is received over the distal end of the stem 14. The sleeve 26 mates with the sleeve mating surface 44 proximally along the stem. The taper between the sleeve 26 and the stem 14 fits such that forces directed distally along the stem (such as patient weight) would push against the taper junction between the stem 14 and sleeve 26. The sleeve 26 generally aligns along the long axis of the stem 14.

The stem 14 receives the neck 32 in a pocket on the head portion of the stem. In a neck neutral orientation, the taper of the neck 32 to the stem 14 may be aligned with the taper of the neck 32 to the femoral head. As the offset, version and height change, the relative direction of the tapers (and thus the forces associated with those tapers) move relative to one another.

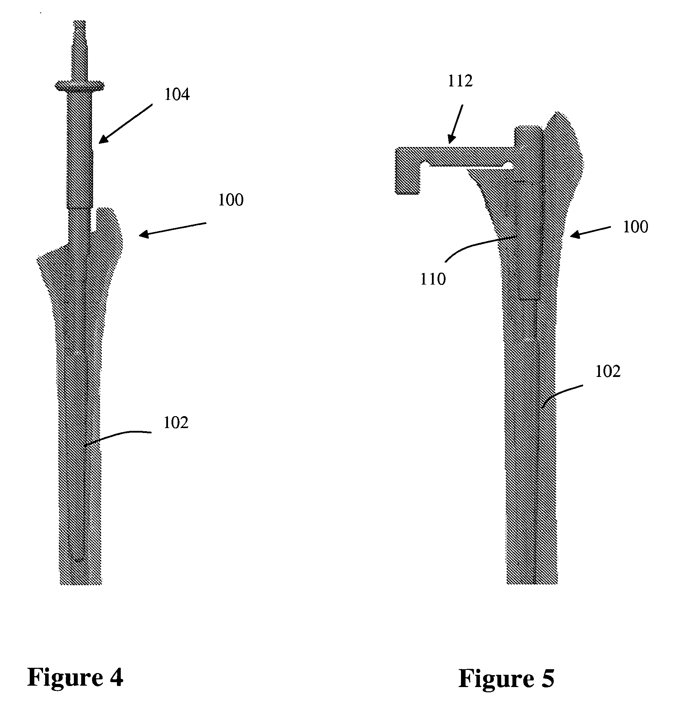

Instruments associated with the implants may provide surgical efficiency during the implantation of the stem. Figures 4 through 11 show steps of a surgical procedure which may be used to prepare, trial, and implant a modular revision hip prosthetic device within a femur. With respect to Figure 4, Figure 4 is a cutaway view of a femur 100 with distal preparation instrumentation according to an aspect of the invention. A distal reamer 102 allows the surgeon to properly prepare and size the canal for optimal fit of the tapered, fluted distally fixed stem. The distal portion of the femur is reamed by attaching a quick connect instrument 104 to an appropriate distal reamer 102 and ream the distal femoral canal in 0.5mm increments until desired distal fit is achieved. It is preferred to begin reaming with a distal reamer that is at least 2mm smaller than the template size. Such a reamer would have little or no resistance to reaming as it progresses through the IM canal. Additionally, to minimize potential risk of reaming through the anterior cortex, the reamer 102 may be directed from anterior to posterior. Preferably, the size of the stem is 0.25mm greater than the equivalently sized reamer, which may provide an automatic press fit.

The depth of the reamer 102 may be determined by aligning a mark on the quick connect instrument with the greater trochanter. If the greater trochanter is not available then alternative anatomical reference may be made. For example, a ruler can be used to measure from the distal end of the osteotomy to the previous location of the greater trochanter. Upon achieving desired distal fit, the quick connect device 104 may be disengaged and the final distal reamer 102 may be left in the canal.

As shown in Figure 5, Figure 5 is a cutaway view of the femur 100 with trialing instrumentation according to an aspect of the invention. When used in conjunction with trial bodies and necks, the distal reamer 102 may function as intramedullary trials which should provide the surgeon with more accurate assessment of implant positioning while reducing the number of instruments and trial components.

In some revision cases, medial bone may need to be removed to accommodate the neck platform of the neck body. An osteotomy jig 112 may be used to address this bone removal. The osteotomy jig 112 is placed over the distal reamer 102 and proximal spacer 110 and may be fixed to reamer 112 with a screw. Arrows on the osteotomy jig 112 may indicate the location where the minimum bone must be removed for the trial neck body (and the subsequent implant) to properly seat. The location of the depth may be marked with a cautery pin. After the jig 112 is removed, an oscillating saw is used to osteotomize the bone. Once the bone is properly oriented, then a trial body may be used to position the body of the stem.

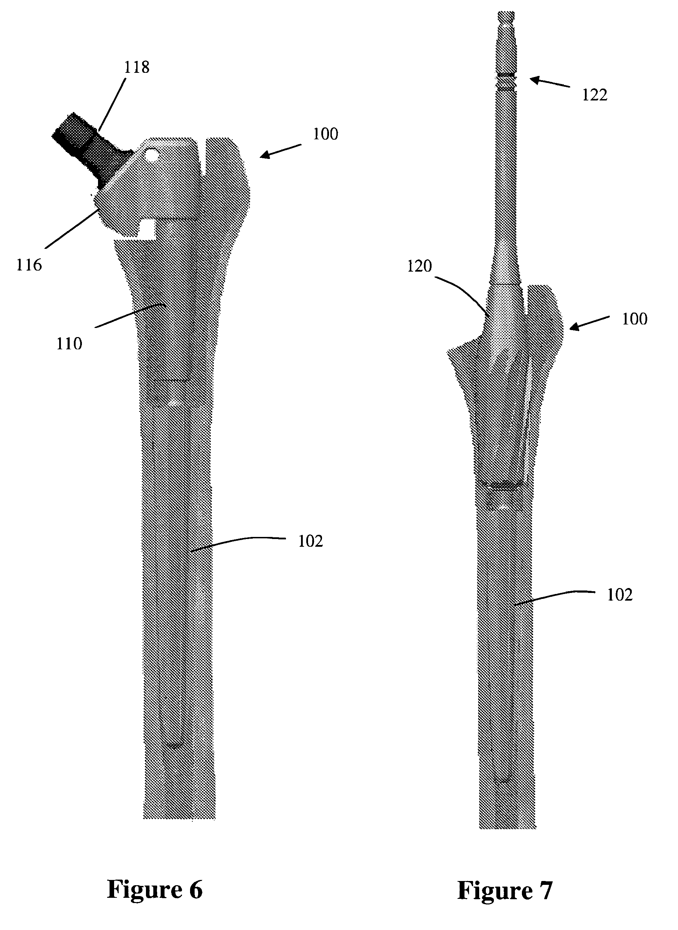

With respect to Figure 6, Figure 6 is a cutaway view of the femur 100 with additional trialing instrumentation according to an aspect of the invention. A trial body 116 is seated on the spacer 110 on the distal reamer 102. A trial neck component 118 is engaged with the body 116. Indicia on the trial neck 116 may orient the neck 118 relative to the body 116. With the trial completely formed, a range of motion (ROM) exercise may be performed to confirm proper seating of implant, assess joint tension, and ensure there is no impingement to the hip. Trial neck components 118 and trial neck pocket version (manipulated by adjusting the rotation of the body 116 on the spacer 110) may be adjusted as appropriate to achieve desired leg length, neck offset, and neck version. A mark can be made on the bone with a cautery pin to mark desired version for implant.

Turning now to Figure 7, Figure 7 is a cutaway view of the femur 100 with proximal preparation instrumentation according to an aspect of the invention. A proximal reamer120 may be used over the distal reamer 102. These "over the top" reamers 120 allow the surgeon to leave the distal reamer 102 in the canal to maintain the relative position of the cavities for the proximal sleeve and the distal taper of the stem. By reaming "over the top" the surgeon is preparing for the proximal segment based on the location of the distal reamer.

The distal reamer may be used by first removing the trial neck body, trial neck component, and proximal trial spacer. The distal reamer 102 is left in the canal. Beginning with the smallest appropriate size, the proximal cone reamer 120 is advanced over the shaft of the distal reamer 102. The proximal cone reamer 120 will rotate, however the distal reamer 102 will act as a guide and should not advance. If an ETO is not performed or if the femoral canal is small, a starter proximal reamer may be necessary to remove bone that may impede the application of the proximal cone reamers 120. The canal is progressively reamed using the proximal cone reamers until the desired proximal fit is achieved.

Following the proximal sleeve preparation, re-trialing may be performed using the proximal trial spacer, trial neck body, trial neck, and trial head component over the distal reamer. If re-trialing is not performed following the proximal sleeve preparation, then the distal reamer component may be removed.

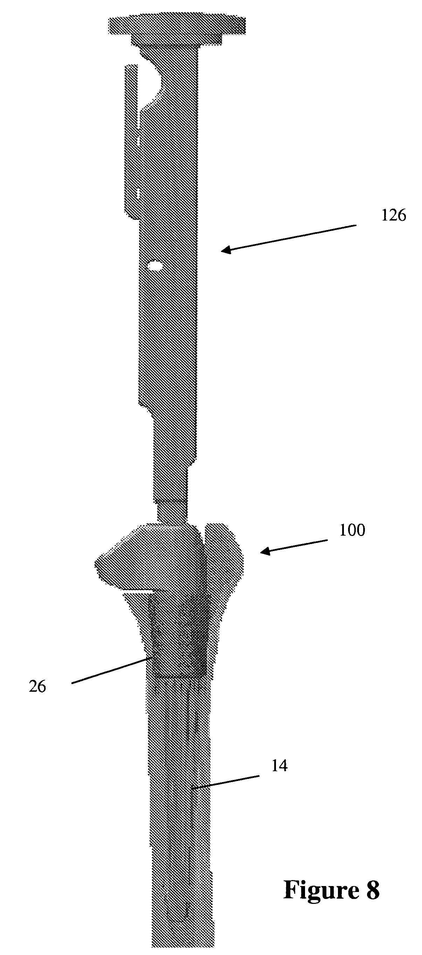

With respect to Figure 8, Figure 8 is a cutaway view of the femur 100 with portions of a hip implant and implant instrumentation. The stem 14 and sleeve 26 are prepared for implant. The sleeve 26 is slid over the distal end of the stem 14 and seated on the stem 14. A stem insertion instrument 126 attaches to the proximal end of the stem 14 generally along the long axis of the stem. The stem/sleeve assembly is impacted into the femur using a mallet against the driving platform. The stem inserter should not impinge on the trochanter. This may cause inadequate stem seating, trochanteric fracture or varus positioning. The surgeon may assess height and neck pocket orientation as the stem advances into the desired position. If a cautery pin mark was made during the trialing step, it can be used as a reference. The stem inserter instrument 126 is then disengaged when the stem is properly seated in the canal.

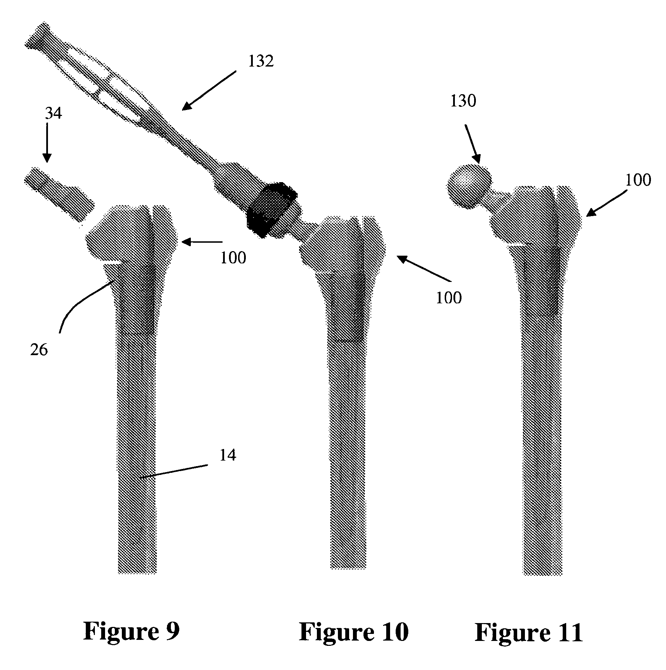

Turning now to Figures 9 through 11, Figures 9 through 11 are cutaway views of the femur 100 with portions of a hip implant 14, 26 and 34 and hip instrumentation 132 according to an aspect of the invention. Once the stem 14 is seated, it is recommended that re-trialing be performed using the trial neck and head components. Fine-tuning of offset, leg length, and version can be made with the modular neck options. Once proper neck orientation is determined, the modular neck trial is replaced with the modular neck implant 34 prior to impaction. All taper surfaces should be protected, clean and dry prior to assembly to ensure a good taper lock. Once the neck 34 is verified in the desired orientation prior to final impaction, the surgeon impacts the final neck 34 and head implant component 130 simultaneously with the head/ neck impaction tool 132. Correct selection of the neck length and cup, and stem positioning are important. Muscle looseness and/or malpositioning of components may result in loosening, subluxation, dislocation, and/ or fracture of the component and/or bone. A final ROM check may ensure desired implant outcome prior to closing with the implants implanted.

In view of the foregoing, it will be seen that the several advantages of the invention are achieved and attained.

The embodiments were chosen and described in order to best explain the principles of the invention and its practical application to thereby enable others skilled in the art to best utilize the invention in various embodiments and with various modifications as are suited to the particular use contemplated.

As various modifications could be made in the constructions and methods herein described and illustrated without departing from the scope of the invention, it is intended that all matter contained in the foregoing description or shown in the accompanying drawings shall be interpreted as illustrative rather than limiting. Thus, the breadth and scope of the present invention should not be limited by any of the above-described exemplary embodiments, but should be defined only in accordance with the following claims appended hereto.

## Claims

1. A revision hip implant, comprising:
a. a stem (10) having a proximal body portion (42), a proximal intramedullary canal portion (44) and a distal intramedullary canal portion (40), the proximal intramedullary canal portion (44) and the distal intramedullary canal portion (40) being generally aligned along a long axis of the stem, the proximal body portion (42) being proximally oriented relative to the proximal intramedullary canal portion (44) and further having a surface (52) extending generally radially out from the long axis of the stem, the proximal intramedullary canal portion (44) having a distally extending taper surface (50);
b. a sleeve (20) configured to be received over the distal intramedullary canal portion (40) of the stem and further having an inner surface (60) configured to mate with the distally extending taper surface (50) of the proximal intramedullary canal portion (44); and
c. a neck (30) configured to be received in the proximal body portion (42) of the stem, the proximal body portion (42) of the stem having a neck receiving surface (54) configured to mate with a neck taper portion (80) of the neck, the neck taper portion (80) being oriented at an angle relative to the long axis;
the sleeve (20) further comprising a conical portion (66) and a cylindrical portion (68), the conical portion (66) being oriented proximal to the proximal stem body portion (42) and the cylindrical portion (68) extending distally away from the conical portion (66).

2. The revision hip implant of claim 1, wherein the distal intramedullary portion (40) of the stem further comprises flutes (48) extending along the length of the stem.

3. The revision hip implant of any of the above claims, wherein the surface (52) extending generally radially out from the long axis of the stem on the proximal body portion (42) of the stem is configured to rest upon a bone cut.

4. The revision hip implant of any of the above claims, wherein the sleeve (20) is configured to asymmetrically extend away from the long axis of the stem.

5. The revision hip implant of any of the above claims, wherein the neck (30) is received on the stem along a first axis, a head (130) is received on the neck along a second axis, the first and second axes being parallel to each other.

6. The revision hip implant of any of the above claims, wherein the stem (10), sleeve (20) and neck (30) are each respectively chosen from groups of differently sized and shaped stems, sleeves and necks.

## Patentansprüche

1. Ein Hüftrevisionsimplantat, das Folgendes beinhaltet:
a. einen Schaft (10) mit einem proximalen Körperabschnitt (42), einem proximalen intramedullären Kanalabschnitt (44) und einem distalen intramedullären Kanalabschnitt (40), wobei der proximale intramedulläre Kanalabschnitt (44) und der distale intramedulläre Kanalabschnitt (40) im Allgemeinen entlang einer Längsachse des Schafts ausgerichtet sind, wobei der proximale Körperabschnitt (42) relativ zu dem proximalen intramedullären Kanalabschnitt (44) proximal orientiert ist und ferner eine Oberfläche (52) aufweist, die sich im Allgemeinen radial von der Längsachse des Schafts aus nach außen erstreckt, wobei der proximale intramedulläre Kanalabschnitt (44) eine sich distal erstreckende kegelförmige Oberfläche (50) aufweist;
b. eine Hülse (20), die so konfiguriert ist, dass sie über dem distalen intramedullären Kanalabschnitt (40) des Schafts aufgenommen wird, und ferner eine Innenoberfläche (60) aufweist, die so konfiguriert ist, dass sie mit der sich distal erstreckenden kegelförmigen Oberfläche (50) des proximalen intramedullären Kanalabschnitts (44) zusammenzupasst; und
c. einen Hals (30), der so konfiguriert ist, dass er in dem proximalen Körperabschnitt (42) des Schafts aufgenommen wird, wobei der proximale Körperabschnitt (42) des Schafts eine Hals aufnehmende Oberfläche (54) aufweist, die so konfiguriert ist, dass sie mit einem kegelförmigen Halsabschnitt (80) des Halses zusammenzupasst, wobei der kegelförmige Halsabschnitt (80) in einem Winkel relativ zu der Längsachse orientiert ist;
wobei die Hülse (20) ferner einen konischen Abschnitt (66) und einen zylindrischen Abschnitt (68) beinhaltet, wobei der konische Abschnitt (66) zu dem proximalen Schaftkörperabschnitt (42) proximal orientiert ist und sich der zylindrische Abschnitt (68) distal von dem konischen Abschnitt (66) aus weg erstreckt.

2. Hüftrevisionsimplantat gemäß Anspruch 1, wobei der distale intramedulläre Abschnitt (40) des Schafts ferner Auskehlungen (48) beinhaltet, die sich entlang der Länge des Schafts erstrecken.

3. Hüftrevisionsimplantat gemäß einem der vorhergehenden Ansprüche, wobei die Oberfläche (52), die sich im Allgemeinen an dem proximalen Körperabschnitt (42) radial von der Längsachse des Schafts aus nach außen erstreckt, , so konfiguriert ist, dass sie auf einem Knochenschnitt aufliegt.

4. Hüftrevisionsimplantat gemäß einem der vorhergehenden Ansprüche, wobei die Hülse (20) so konfiguriert ist, dass sie sich asymmetrisch von der Längsachse des Schafts weg erstreckt.

5. Hüftrevisionsimplantat gemäß einem der vorhergehenden Ansprüche, wobei der Hals (30) auf dem Schaft entlang einer ersten Achse aufgenommen wird, ein Kopf (130) auf dem Hals entlang einer zweiten Achse aufgenommen wird und die erste und zweite Achse parallel zueinander sind.

6. Hüftrevisionsimplantat gemäß einem der vorhergehenden Ansprüche, wobei der Schaft (10), die Hülse (20) und der Hals (30) jeweils aus Gruppen mit unterschiedlich bemessenen und ausgebildeten Schäften, Hülsen bzw. Hälsen ausgewählt sind.

## Revendications

1. Un implant de hanche de révision, comprenant :
a. une tige (10) possédant une partie de corps proximale (42), une partie de canal intramédullaire proximale (44) et une partie de canal intramédullaire distale (40), la partie de canal intramédullaire proximale (44) et la partie de canal intramédullaire distale (40) étant globalement alignées suivant un axe long de la tige, la partie de corps proximale (42) étant orientée de manière proximale par rapport à la partie de canal intramédullaire proximale (44) et possédant en sus une surface (52) s'étendant globalement de manière radiale vers l'extérieur depuis l'axe long de la tige, la partie de canal intramédullaire proximale (44) possédant une surface biseautée s'étendant de manière distale (50) ;
b. un manchon (20) configuré pour être reçu sur la partie de canal intramédullaire distale (40) de la tige et possédant en sus une surface intérieure (60) configurée pour s'apparier à la surface biseautée s'étendant de manière distale (50) de la partie de canal intramédullaire proximale (44) ; et
c. un col (30) configuré pour être reçu dans la partie de corps proximale (42) de la tige, la partie de corps proximale (42) de la tige possédant une surface de réception de col (54) configurée pour s'apparier à une partie biseautée de col (80) du col, la partie biseautée de col (80) étant orientée selon un angle par rapport à l'axe long ;
le manchon (20) comprenant en sus une partie conique (66) et une partie cylindrique (68), la partie conique (66) étant orientée de manière proximale à la partie de corps de tige proximale (42) et la partie cylindrique (68) s'étendant de manière distale en s'éloignant de la partie conique (66).

2. L'implant de hanche de révision de la revendication 1, dans laquelle la partie intramédullaire distale (40) de la tige comprend en sus des cannelures (48) s'étendant sur la longueur de la tige.

3. L'implant de hanche de révision d'une quelconque des revendications précédentes, dans laquelle la surface (52) s'étendant globalement de manière radiale vers l'extérieur depuis l'axe long de la tige sur la partie de corps proximale (42) de la tige est configurée pour reposer sur une section d'os.

4. L'implant de hanche de révision d'une quelconque des revendications précédentes, dans laquelle le manchon (20) est configuré pour s'étendre de manière asymétrique en s'éloignant de l'axe long de la tige.

5. L'implant de hanche de révision d'une quelconque des revendications précédentes, dans laquelle le col (30) est reçu sur la tige le long d'un premier axe, une tête (130) est reçue sur le col le long d'un deuxième axe, les premier et deuxième axes étant parallèles l'un à l'autre.

6. L'implant de hanche de révision d'une quelconque des revendications précédentes, dans laquelle la tige (10), le manchon (20) et le col (30) sont chacun choisis respectivement dans des groupes de tiges, manchons et cols de formes et tailles différentes.
